# EUROPÄISCHE PATENTSCHRIFT

(11) **EP 3 311 883 B1**
(45) Veröffentlichungstag und Bekanntmachung des Hinweises auf die Patenterteilung: **29.01.2020**
(21) Anmeldenummer: 16195002.7
(22) Anmeldetag: 21.10.2016
(51) Int. Cl.: A61N 1/375, H01B 1/02

(54) **ANSCHLUSSSTIFT UND DURCHFÜHRUNG**
TERMINAL PIN AND FEEDTHROUGH
BROCHE DE RACCORDEMENT ET TRAVERSÉE

(43) Veröffentlichungstag der Anmeldung: 25.04.2018
(73) Patentinhaber: BIOTRONIK SE & Co. KG, 12359 Berlin (DE)
(72) Erfinder: Eigenbrod, Hella, 90530 Wendelstein (DE); Fries, Lilli, 90547 Stein (DE)
(74) Vertreter: Galander, Marcus

(56) Entgegenhaltungen:
- EP-A1- 3 072 553
- US-A1- 2016 104 947
- US-B2- 7 340 305

## Beschreibung

Die Erfindung betrifft einen Anschlussstift zum elektrischen Anschluss eines Leitungsträgers, der zur stoffschlüssigen Anbindung an eine Leiterfläche des Leitungsträgers vorgesehen ist. Sie betrifft des Weiteren eine Durchführung eines implantierbaren elektromedizinischen Gerätes (IMD).

Die meisten praktisch bedeutsamen implantierbaren medizinischen Geräte (IMDs) sind dazu vorgesehen, über geeignet platzierte Elektroden elektrische Impulse an reizbares Körpergewebe abzugeben. Ferner können viele Geräte elektrische Impulse und Reize im Körper des Patienten gezielt messen und über einen längeren Zeitraum aufzeichnen oder auswerten, um eine individuell angepasste Therapie zu wählen und den Erfolg der Behandlung in vivo zu überprüfen.

Um diese Funktionen auszuführen, sind im Gehäuse des Gerätes elektronische/elektrische Funktionseinheiten zur Erzeugung und Messung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Diese Aufgabe wird von sogenannten Durchführungen übernommen, die Gegenstand zahlreicher und höchst unterschiedlicher Entwicklungen gewesen sind. Die Aufgabe einer Durchführung besteht darin, die elektrischen Signale durch das hermetisch geschlossene Gehäuse zu führen und so einen elektrischen Kontakt der Elektronik im hermetisch dichten Gehäuse mit den Elektroden im Körper des Patienten zu ermöglichen. In vielen solchen Durchführungen werden hierfür Anschlussstifte eingesetzt, die im Geräteinneren mit der dort befindlichen Leiterplatte oder einem ähnlichen Leitungsträger kontaktiert werden und die Signale durch das Gehäuse führen.

Die US 7,747,321 B2 offenbart, wie in Fig. 1 gezeigt, einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet. Sie umfassen einen Draht-Kern, etwa aus Tantal, Niob, Titan, Molybdän oder Kupfer und eine oxidationsbeständige Ummantelung aus einem biokompatiblem Material, etwa Gold, Platin, Titan oder ähnlichem.

In bekannten Durchführungskonstruktionen werden als Anschlusselemente Stift-Plättchen-Konstruktionen benutzt, bei denen ein langgestrecktes Anschlusselement (Stift) einen Isolationskörper der Durchführung durchstößt und zum Anschweißen an ein Kopfteil des IMD genutzt wird, während ein auf das gehäuse-seitige Ende des Stiftes aufgelegtes Plättchen den Kontakt zur Geräteelektronik (Leiterplatte) herstellt. Der Stift kann aus Materialien wie Nb oder PtIr und das Plättchen aus Cu oder Ni bestehen, der Stift ist im Isolationskörper hart-verlötet, und das Plättchen ist typischerweise ebenfalls hart auf das Ende des Stiftes gelötet oder über einen Laserschweißpunkt mit ihm verbunden. Das Plättchen ist üblicherweise durch eine Lotschicht (Vorverzinnung) oder mit einer dünnen Pd-Schicht vor Oxidation geschützt.

Aus der US 7,340,305 B2 ist eine entsprechende Durchführung eines IMD bekannt, in der Anschlussstifte eingesetzt werden, die einen nicht aus teurem Platin, Platin/Iridium oder Palladium bestehenden Kern und eine leitfähige Beschichtung haben. Die Beschichtung ermöglicht es einerseits, eine Oxidation des Anschlussstifts unter den Einsatzbedingungen eines implantierten Gerätes zu steuern bzw. zu begrenzen, und andererseits ermöglicht sie es, den Anschlussstift durch ein Weichlötverfahren mit einer Leiterplatte zu verbinden. Auch die US 2011/0303458 A1 offenbart Anschlussstifte mit partiell mehrschichtigem Aufbau und deren Einsatz bei Durchführungen von IMDs.

Aus der US 2016/0104947 A1 ist eine weitere mehrteilige Anschlusselement-Konstruktion einer Durchführung eines IMD bekannt, bei der auf den Anschlussstift anstelle eines Plättchens eine Kappe aus einem gegenüber dem Stift härteren Material aufgesetzt ist, die anschließend in einem Tauchbad mit Lötmittel beschichtet wird.

Im Zuge der Miniaturisierung und der Fortschritte der Elektronik hat sich die Weichlöttechnik (Solder) in den letzten Jahren rasant gewandelt. Manuelle Bestückungsprozesse wurden zunehmend von vollautomatischen Pick&Place-Automaten verdrängt und die Through Hole Technology (THT) wurde allmählich durch die Oberflächenmontage (SMT) ersetzt. Dies ermöglicht kleinere, kompaktere Schaltungen und führt somit schrittweise auch zu kleineren, patientenverträglicheren IMDs. Um die vielen Vorteile der SMT erreichen zu können, müssen Durchführungen die Anforderungen an ein oberflächenmontiertes Bauteil (SMD) genügen.

Die o. a. bekannten Lösungen erfordern einen hohen Montage- und insgesamt Fertigungsaufwand. Es werden Komponenten erstellt, die nach oder während dem Hochtemperaturlöten (brazing) mit der Durchführung gefügt werden. Der zusätzliche Bestückungsaufwand ist nicht unerheblich. Er kann dazu führen, dass die Fertigungskosten für die zusätzlichen Komponenten deren Materialwert um ein Vielfaches übersteigen. Die zusätzlichen Komponenten sind meist sehr klein und grazil (typischerweise <1 mm) und daher schwierig zu platzieren und auszurichten. Weiterhin werden separate Fertigungshilfsmittel zur Montage der zusätzlichen Komponenten benötigt. Die Komponenten müssen vor der Montage extra gelagert und geprüft werden und benötigen zusätzliche Chargenführung und -kontrolle.

Durch den zusätzlichen Fügeprozess zwischen Stift und Zusatzelement (Plättchen) entsteht potenziell Ausschuss. Findet der Fügeprozess des Stiftes aus Komponenten integriert mit dem Fügeprozess der Durchführung statt, so muss eine zusätzliche Sortierprüfung durchgeführt werden, bei der der Prozess des Stiftfügens beurteilt wird. Nach dem Fügen muss die Verbindungsstelle auf Haftung, ausreichende Stabilität und Lebensdauer untersucht werden. Grundsätzlich können eine Verkippung und ein Versatz der Bauteile des Stiftes und dadurch resultierend ein Versatz beim Platzieren auf der Leiterplatte auftreten. Dieser muss in einem gesonderten Prüfschritt festgestellt und bei der Durchführungs-Montage vermieden und geprüft werden.

Das Dokument EP 3 072 553 A1 offenbart einen Anschlussstift zum elektrischen Anschluss eines Leitungsträgers, wobei der Anschlussstift zur stoffschlüssigen Anbindung an eine Leiterfläche des Leitungsträgers vorgesehen ist, aus einem biokompatiblen Nichtedelmetall gebildet ist, an oder nahe einem Ende einen gegenüber einem end-ferneren Abschnitt verdickten Abschnitt hat und mindestens ein Teil der Oberfläche des verdickten Abschnitts eine weichlötbare Beschichtung mit einem Edelmetall aufweist. Über oder unter der weichlötbaren Beschichtung kann eine Diffusionsbarriere gebildet sein, welche die Metalle Aluminium, Nickel, Molybdän oder Gold aufweist. Des Weiteren kann der Anschlussstift einen Schutzfilm zur Verhinderung von Oxidation aufweisen.

Aufgabe der Erfindung ist es, einen gegenüber bekannten Anordnungen verbesserten Anschlussstift bereitzustellen, der insbesondere kostengünstig herzustellen und zu verarbeiten und dennoch von hoher Qualität ist und dessen Einsatz einen verringerten Prüf- und Montageaufwand bei der Montage (SMT) von Durchführungen ermöglicht.

Diese Aufgabe wird durch einen Anschlussstift mit den Merkmalen des Anspruchs 1 gelöst. Zweckmäßige Fortbildungen des Erfindungsgedankens sind Gegenstand der abhängigen Ansprüche. Mit der Erfindung wird des Weiteren eine Durchführung mit den Merkmalen des Anspruchs 10 bereitgestellt.

Erfindungsgemäß ist vorgesehen, dass der Anschlussstift ein verdicktes Ende hat und mindestens ein Teil der Oberfläche des verdickten Endes eine weichlötbare Beschichtung mit mindestens zwei übereinanderliegenden funktional differenzierten Teilschichten aufweist. Dies bewirkt, dass der vorgeschlagene Anschlussstift auf einer gewöhnlichen SMD-Leiterplatte oder einem ähnlichen Leitungsträger mittels eines etablierten Weichlotverfahrens gefügt werden kann, wobei das Lötverfahren kompatibel zu etablierten Prozessen und Materialien ist und eine langzeitstabile Verbindung liefert. Die mehrteilige Beschichtung des Anschlussstiftes ermöglicht die Optimierung des Qualitäts-/Kosten-Verhältnisses, der Lagerfähigkeit und der Handling-Eigenschaften.

Gegenüber etablierten Konfigurationen, die mehrteilige Anschlussstifte beinhalten, ergeben sich folgende spezielle Vorteile:
- Integration mehrerer Komponenten in eine Komponente
- Kostengünstigere Produktion ohne Redesign der Produkte bzw. Produktneuentwicklung
- Geringere Ausschusszahlen
- Weniger Bestückungsaufwand mit der Folge niedrigerer Kosten
- Höhere Maß- und Positionsgenauigkeit.

In einer zweckmäßigen Ausführung der Erfindung ist die Beschichtung so aufgebaut, dass sie auf einem Anschlussstift-Körper die Haftvermittlerschicht und mindestens mittelbar über dieser die Lötverbindungsschicht umfasst und mindestens mittelbar über dieser die Oxidationsschicht.

Generell kann die Beschichtung die gesamte Oberfläche des verdickten Endes bedecken, was eine technologisch besonders einfache Erzeugung der Beschichtung ermöglicht. Andererseits kann es zweckmäßig sein, dass die Beschichtung nur den Boden und optional den Umfang oder Teilabschnitte des Umfangs des nagelkopf-artigen verdickten Endes bedeckt. Eine Beschichtung kann zur vorteilhaften Ausführung eines direkten Weichlötens des Anschlussstiftes mit dem Leitungsträger oder auch zum Bonden von Anschlussdrähten an den Anschlussstift führen.

Die Lötverbindungsschicht kann mindestens ein Edelmetall oder eines der Metalle Nickel, Kupfer, Silber, Zinn oder eine Legierung mit mindestens eines dieser Metalle enthalten. Typische Schichtdicken betragen einige Mikrometer. Es sind auch verwandte Metalle einsetzbar, so etwa eines oder mehrere der Elemente Iridium, Rhodium oder Ruthenium. Es versteht sich, dass auch verschiedenste Legierungen von mindestens zwei der hier erwähnten Elemente, vorteilhaft insbesondere kommerziell relativ kostengünstig verfügbare Legierungen, bei der Ausführung der Erfindung verwendbar sind. Als Material des unbehandelten Anschlussstiftes bzw. des Stift-Grundkörpers kommen insbesondere Kupfer oder Titan, aber auch biokompatible Elemente wie Tantal, Niob oder Molybdän und auch wiederum Legierungen dieser in Betracht. Die Haftvermittlerschicht weist eine der Verbindungen Niobnitrid, Nickelnitrid, Titannitrid, oder Kupfernitrid auf.

Alternativ kann der Anschlussstift aus einem kostengünstigen Substrat (z. B. Kupfer, Konstantan, Nickel) gefertigt sein. In der Ausführung, dass es sich um nicht biokompatibles Material handelt, wird der Stift nach dem formgebenden Verfahren mit einem biokompatiblen Material (z. B. Titan, Tantal, Niob, Platin, Palladium oder Legierungen daraus) beschichtet. Die Beschichtung muss mindestens den Teil umfassen, welcher sich nicht innerhalb des späteren IMDs Gehäuse befindet.

In einer weiteren Ausführung, die insbesondere im Kontext des vorgenannten Aspekts der Erfindung steht, ist eine Teilschicht der Beschichtung als Oxidationsschutzschicht ausgebildet, und diese weist insbesondere mindestens eines der Edelmetalle, wie Gold, Platin, Palladium oder ähnliches, auf. Die Oxidationsschutzschicht kann über oder unter die weichlötbare Beschichtung aufgebracht werden. Es kann auch sinnvoll sein, mehrere übereinander aufgebrachte Oxidationsschutzschichten zu erzeugen und diese zu kombinieren oder übereinander zu schichten, um eine besonders hohe Langlebigkeit der mit einem solchen Anschlussstift gebildeten Durchführung zu erreichen.

Gemäß einer technologischen Ausgestaltung der Erfindung ist die Beschichtung oder mindestens eine Schicht hiervon als galvanische Beschichtung oder als durch ein Vakuum-Beschichtungsverfahren erzeugte Dünnschicht ausgeführt. Zweckmäßige Schichtdicken liegen zwischen 0,01 µm (für ein Dünnschichtverfahren) und 10 µm (für ein Galvanikverfahren). Beide Ausführungen haben spezifische Vor- und Nachteile; beispielsweise ist die Schichtbelegung bei der Galvanik sehr selektiv, die Schicht jedoch häufig mit Fremdstoffen aus dem Galvanikbad kontaminiert. Das Sputtern unter Vakuum erfolgt allseitig und mit sehr hoher Reinheit.

Zum Schutz der Beschichtung vor Verunreinigung, Kontamination und Oxidation kann die Beschichtung auf dem Stift bis zur weiteren Verarbeitung versiegelt werden. Hierfür kann ein Polymer oder ein organischer Schutzfilm verwendet werden (OSP - Organic Surface Protection). Bekannte Schutzfilme (z. B. Glicoat, ENTEK+) können vollständig oder selektiv auf die weichlötbare Schicht oder den ganzen Stift aufgebraucht sein. Typische Schichtdicken betragen 0,2 µm bis 0,6 µm und enthalten zum Beispiel substituierte Imidiazole und/oder Triazole. Der Schutzfilm verhindert die Oxidation des Grundmaterials während der Lagerung typischerweise für mehrere Monate und pyrolysiert unmittelbar vor bzw. während Weichlötprozesses. Verbrennungsrückstände auf den Platinen können während der etablierten Waschprozesse in automatisierten Anlagen rückstandsfrei entfernt werden. Weitere Möglichkeiten bestehen in der Beschichtung durch ein Element der Edelmetallgruppen.

In weiteren Ausführungen der Erfindung ist der verdickte Endabschnitt rotationssymmetrisch am Ende des Anschlussstiftes im Wesentlichen in Art eines Nagelkopfes gebildet. Diese Ausführung ist mit seit langem etablierten Stauchverfahren sehr leicht und kostengünstig herzustellen und bietet, je nach konkreter Ausführung, die Möglichkeit einer besonders einfachen Anpassung der Endfläche des Anschlussstiftes an die für die Verbindung mit der entsprechenden Leiterbahn verfügbare Grundfläche.

Die Herstellung des verdickten Stiftendes (Nailheads) kann durch umformende Verfahren geschehen, hierzu wird ein gezogener Draht aus Nb/PtIr/Ni/Ta/Cu o. ä. auf die gewünschte Länge zugeschnitten. Anschließend wird an einem Ende ein Kopf angestaucht. Die Form des Nailheads kann weiterhin durch urformende Verfahren, d. h. durch Gießen oder alternative Fertigungsverfahren, oder durch spanende Formgebung hergestellt werden.

In weiteren Ausgestaltungen wird die Formgebung am Ende des Stiftes der Kontaktfläche (Land Pattern) der Leiterplatte (PCB) angepasst. Bekannte und häufig verwendete Grundflächen sind hier Rechtecke, Sechsecke und Achtecke oder andere Polygone. Um die leichte Herstellbarkeit gewährleisten zu können, müssen die Ecken des Umformwerkzeuges verrundet werden, damit der Stift nach dem Umformen ausgeformt werden kann. Die Kontaktfläche zwischen Durchführungsstift und Leiterplatte ist vergrößert, wodurch der Übergangswiderstand gesenkt und die Belastbarkeit der Fügeverbindung zwischen Stift und Platine vergrößert werden können.

In weiteren Ausführungen ist am Ende des Anschlussstiftes ein insbesondere kegelförmiger oder halbkugelförmiger Abschnitt ausgeformt, der sich zum äußersten Ende hin verjüngt, oder auch ein ballonartiger "Aufsatz". Vorteilhaft ist eine solche zusätzliche Geometrie an der Unterseite des Stiftes, welche die Funktion einer Auflagefläche auf der Platine übernimmt, aus dem folgenden Grund: Der Stift sollte während des Weichlötprozesses nicht vollflächig mit der Platine abschließen. Es bildet sich ein definierter Lötspalt zwischen Stift und Platine in den flüssiges Weichlot eindringen kann bzw. in dem Lötpaste beim Aufsetzten der Durchführung in die Lötpaste (solder paste) und der anderen der Bauteile nicht komplett verdrängt wird bzw. an definierten Stellen verbleibt.

Vorteilhaft kann es im Übrigen sein, die Stiftenden spitz oder scharfkantig ausführen, damit diese sich durch definiertes Andrücken im Substratmaterial der Leiterplatte (PCB) zentrieren bzw. fixieren können. So ist gewährleistet, dass die Durchführung während des Durchfahrens im Reflowofen in der Lötpaste nicht aufschwimmt oder verrutscht. Lötfehler wie Seiten- bzw. Spitzenüberhang können so effektiv vermindert werden. Der Prozess kann hierdurch mit üblichen Prozessparametern ablaufen, auch wenn der Masseschwerpunkt bzw. das Trägheitsmoment der Durchführung designbedingt untypisch für SMD-Bauteile liegen.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische Darstellung eines herkömmlichen Herzschrittmachers und
- Fig. 2 bis 7: jeweils Längsschnittdarstellungen von Anschlussstiften gemäß Ausführungsbeispielen der Erfindung.

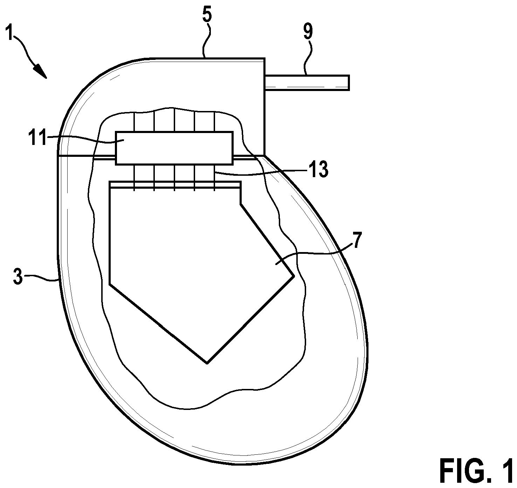

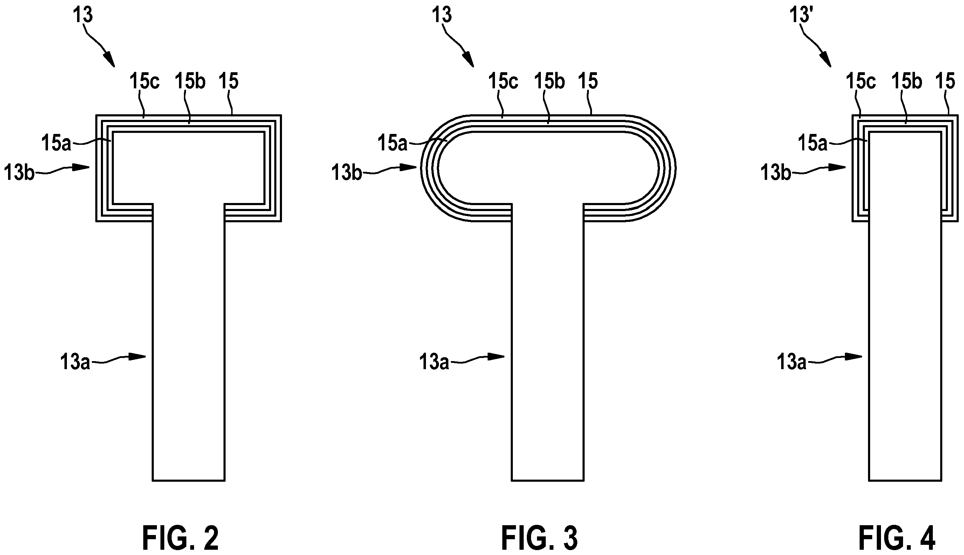

In allen Figuren 2 bis 7 ist der Anschlussstift jeweils, in Anlehnung an Fig. 1 und unabhängig von den abweichenden geometrischen Formen, einheitlich mit den Ziffern 13 bzw. 13' bezeichnet, und die Bezeichnung einzelner Abschnitte oder der jeweiligen Beschichtung ist entsprechend gewählt.

Fig. 2 zeigt einen Anschlussstift 13 mit einem über den größten Teil seiner Erstreckung zylindrischen Grundkörper 13a und einem stufenartig verdickten und ebenfalls zylindrischen Ende 13b. Auf der gesamten Oberfläche des verdickten Endes 13b ist eine Beschichtung 15 aufgebracht, die eine untere Haftvermittlerschicht 15a, eine diese abdeckende Lötverbindungsschicht 15b und eine darüber liegende Oxidationsschutzschicht 15c umfasst. Auf dem Umfang des längeren Stiftabschnitts 13a mit geringerem Durchmesser ist hingegen keinerlei Beschichtung dargestellt; ggfs. kann hier eine das Hartverlöten in einer Durchführung fördernde Beschichtung vorgesehen sein, die jedoch im Kontext der vorliegenden Erfindung irrelevant ist.

Fig. 3 zeigt einen weiteren Anschlussstift 13 mit sehr ähnlichem Aufbau wie dem in Fig. 2 gezeigten, der sich von jenem jedoch durch ein ballonartig abgerundetes verdicktes Ende 13b unterscheidet. Der Aufbau der Beschichtung ist der gleiche wie bei Fig. 2.

Fig. 4 zeigt als weiteres Beispiel einen Anschlussstift 13', der einen zylindrischen Körper 13a mit zwischen beiden Enden konstanten Durchmesser umfasst. An einem der Enden ist eine die Stirnfläche und einen Teil des Umfangs des Grundkörpers 13a umfassende Beschichtung 15 vorgesehen, deren Aufbau demjenigen der Beschichtungen aus Fig. 2 und 3 entspricht. Durch die mehrteilige Beschichtung ergibt sich eine Verdickung des Anschlussstiftes am beschichteten Ende, deren Ausmaß jedoch deutlich geringer als bei den Anschlussstiften nach Fig. 2 und 3 ist.

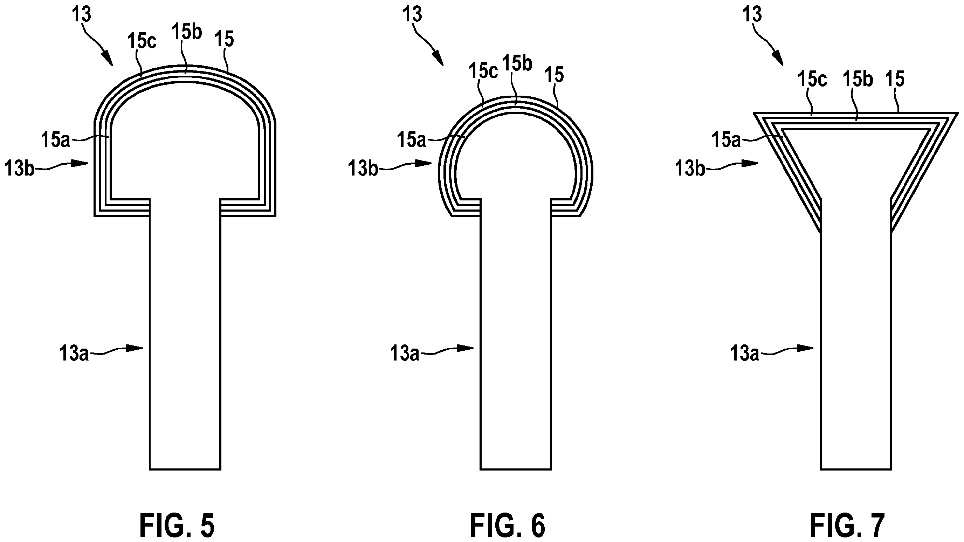

Fig. 5 zeigt als weiteres Beispiel einen Anschlussstift 13, bei dem wiederum - wie in Fig. 2 und 3 - am Ende des zylindrischen Abschnitts 13a eine stufenartige integrale Verdickung 13b aus dem Material des Anschlussstift-Körpers vorgesehen ist. Diese hat hier die Gestalt eines zylindrischen Abschnitts mit ballig ausgeführter freier Stirnfläche bzw. Endfläche. Der Aufbau der Beschichtung 15 entspricht den vorher genannten Beispielen.

Fig. 6 zeigt als weiteres Beispiel einen Anschlussstift 13 mit grundsätzlich gleichem Aufbau wie bei Fig. 2, 3 und 5, jedoch mit einem verdickten Endabschnitt 13b in Form eines Kugelsegments, welches etwas größer als eine Halbkugel ist.

Fig. 7 schließlich zeigt als weiteres Ausführungsbeispiel einen Anschlussstift 13, dessen größerer zylindrischer Teil 13a sich konisch zu einem kegelstumpfförmigen Ende 13b erweitert. Ansonsten ist hier der Aufbau wiederum der gleiche wie bei den vorgenannten Ausführungen.

Die Ausführung der Erfindung ist in vielerlei anderen Ausführungen und in verschiedensten Kombinationen der oben als Merkmale verschiedener Ausführungsformen dargestellten Merkmale möglich, insbesondere auch mit mehr als zweiteiligen bzw. zweischichtigen Beschichtungssystemen, mit anderen als den oben genannten Materialien und in vielerlei geometrischen Ausgestaltungen der jeweiligen Verdickungen bzw. endferneren Bereiche geringeren Durchmessers.

## Patentansprüche

1. Anschlussstift (13; 13') zum elektrischen Anschluss eines Leitungsträgers (7) eines implantierbaren elektromedizinischen Gerätes, wobei der Anschlussstift zur stoffschlüssigen Anbindung an eine Leiterfläche des Leitungsträgers ein verdicktes Ende (13b) hat und mindestens ein Teil der Oberfläche des verdickten Endes eine weichlötbare Beschichtung (15) mit mindestens zwei übereinanderliegenden funktional differenzierten Teilschichten (15a, 15b, 15c) aufweist, von denen eine eine Lötverbindungsschicht (15b) ist und unter der Lötverbindungsschicht (15b) eine Haftvermittlerschicht (15a) vorgesehen ist, **dadurch gekennzeichnet, dass** die Haftvermittlerschicht (15a) eine der Verbindungen Niobnitrid, Titannitrid, Kupfernitrid oder Nickelnitrid aufweist.

2. Anschlussstift nach Anspruch 1, wobei die Beschichtung (15) auf einem Anschlussstift-Körper die Haftvermittlerschicht (15a) und mindestens mittelbar über der Haftvermittlerschicht (15a) die Lötverbindungsschicht (15b) und mindestens mittelbar über der Lötverbindungsschicht (15b) eine Oxidationsschutzschicht (15c) umfasst.

3. Anschlussstift nach Anspruch 1 oder 2, wobei die Beschichtung (13a; 13a') die gesamte Oberfläche des verdickten Endes bedeckt.

4. Anschlussstift nach Anspruch 1 oder 2, wobei die Beschichtung (15) nur den Boden oder den Boden und den Umfang oder Teilabschnitte des Umfangs des verdickten Endes (13b) bedeckt.

5. Anschlussstift nach einem der vorangehenden Ansprüche, wobei die Lötverbindungsschicht (15b) mindestens eines der Metalle Gold, Platin, Kupfer, Silber, Nickel, Iridium und Palladium aufweist.

6. Anschlussstift nach Anspruch 2, wobei der Anschlussstift-Körper (13a, 13b) aus einem Nichtedelmetall gebildet ist und eine direkt das Nichtedelmetall berührende Schicht der Beschichtung (15a) als Haftvermittlerschicht ausgebildet ist.

7. Anschlussstift nach einem der vorangehenden Ansprüche, wobei die Beschichtung (15) oder mindestens eine Schicht hiervon als galvanische Beschichtung oder als durch ein Vakuum-Beschichtungsverfahren erzeugte Schicht ausgeführt ist.

8. Anschlussstift nach einem der vorangehenden Ansprüche, mit mindestens einem Abschnitt, der eine Teilschicht mit einem biokompatiblen Material aufweist, wie Titan, Tantal, Niob, Gold, Platin, Palladium oder einer Legierung mindestens eines dieser Metalle.

9. Anschlussstift nach einem der vorhergehenden Ansprüche, wobei das verdickte Ende (13b) des Anschlussstiftes einen als Zylinder, Kegelstumpf oder Kugelabschnitt oder als vier- oder mehreckiges Prisma oder vier- oder mehrseitiger Pyramidenstumpf im Wesentlichen in Art eines Nagelkopfes gebildeten Erweiterungsabschnitt aufweist.

10. Durchführung (11) für ein implantierbares elektromedizinisches Gerät (1), die mindestens einen Anschlussstift (13a; 13a') nach einem der vorangehenden Ansprüche umfasst.

## Claims

1. A terminal pin (13; 13') for electrically connecting a conductor support (7) of an implantable electromedical device, the terminal pin comprising a thickened end (13b) for the integral attachment to a conductor surface of the conductor support, and at least a portion of the surface of the thickened end comprising a solderable coating (15) having at least two functionally differentiated sub-layers (15a, 15b, 15c) arranged above one another, one of these being a soldered joint layer (15b), and an adhesion promoter layer (15a) being provided beneath the soldered joint layer (15b), **characterized in that** the adhesion promoter layer (15a) comprises one of the compounds niobium nitride, titanium nitride, copper nitride or nickel nitride.

2. The terminal pin according to claim 1, wherein the coating (15) on a terminal pin body comprises the adhesion promoter layer (15a) and, at least indirectly above the adhesion promoter layer (15a), the soldered joint layer (15b) and, at least indirectly above the soldered joint layer (15b), an oxidation protection layer (15c).

3. The terminal pin according to claim 1 or 2, wherein the coating (13a; 13a') covers the entire surface of the thickened end.

4. The terminal pin according to claim 1 or 2, wherein the coating (15) covers only the bottom, or the bottom and the circumference, or portions of the circumference of the thickened end (13b).

5. A terminal pin according to any one of the preceding claims, wherein the soldered joint layer (15b) comprises at least one of the metals gold, platinum, copper, silver, nickel, iridium and palladium.

6. The terminal pin according to claim 2, wherein the terminal pin body (13a, 13b) is formed of a base metal, and a layer of the coating (15a) making direct contact with the base metal is designed as the adhesion promoter layer.

7. A terminal pin according to any one of the preceding claims, wherein the coating (15), or at least one layer thereof, is designed as a galvanic coating or as a film generated by way of a vacuum coating process.

8. A terminal pin according to any one of the preceding claims, comprising at least one section that includes a sub-layer made of a biocompatible material, such as titanium, tantalum, niobium, gold, platinum, palladium or an alloy of at least one of these metals.

9. A terminal pin according to any one of the preceding claims, wherein the thickened end (13b) of the terminal pin comprises an extension section designed as a cylinder, a frustum or a spherical segment or as a quadrangular or polygonal prism or a foursided or multi-sided truncated pyramid, substantially in the form of a nail head.

10. A feedthrough (11) for an implantable electromedical device (1), comprising at least one terminal pin (13a; 13a') according to any one of the preceding claims.

## Revendications

1. Broche de connexion (13 ; 13') permettant la connexion électrique d'un support conducteur (7) d'un appareil électromédical implantable, où la broche de connexion possède une extrémité (13b) renforcée en épaisseur pour une liaison par complémentarité des matières sur une surface conductrice du support conducteur et au moins une partie de la surface de l'extrémité renforcée en épaisseur présente un revêtement (15) pouvant être brasé de manière tendre avec au moins deux couches partielles (15a, 15b, 15c) superposées, différenciées de manière fonctionnelle, parmi lesquelles l'une est une couche de liaison par brasage (15b) et une couche de promoteur d'adhésion (15a) est prévue en-dessous de la couche de liaison par brasage (15b), **caractérisée en ce que** la couche de promoteur d'adhésion (15a) présente un des composés nitrure de niobium, nitrure de titane, nitrure de cuivre ou nitrure de nickel.

2. Broche de connexion selon la revendication 1, dans laquelle le revêtement (15) comprend la couche de promoteur d'adhésion (15a) sur un corps de broche de connexion et au moins indirectement au-dessus de la couche de promoteur d'adhésion (15a) la couche de liaison par brasage (15a), et au moins indirectement une couche de protection contre l'oxydation (15c) au-dessus de la couche de liaison par brasage (15b).

3. Broche de connexion selon la revendication 1 ou la revendication 2, dans laquelle le revêtement (13a; 13a') recouvre la surface totale de l'extrémité renforcée en épaisseur.

4. Broche de connexion selon la revendication 1 ou la revendication 2, dans laquelle le revêtement (15) ne recouvre que le socle, ou le socle et le périmètre, ou des segments partiels du périmètre de l'extrémité (13b) renforcée en épaisseur.

5. Broche de connexion selon l'une des revendications précédentes, dans laquelle la couche de liaison par brasage (15b) présente au moins l'un des métaux or, platine, cuivre, argent, nickel, iridium et palladium.

6. Broche de connexion selon la revendication 2, dans laquelle le corps de broche de connexion (13a, 13b) est fabriqué dans un métal non noble et une couche du revêtement (15a) n'étant pas en contact direct avec le métal non noble est conçue sous forme de couche de promoteur d'adhésion.

7. Broche de connexion selon l'une des revendications précédentes, dans laquelle le revêtement (15) ou au moins une couche de celui-ci est conçu sous forme de revêtement galvanique ou d'une couche créée par un procédé de revêtement sous vide.

8. Broche de connexion selon l'une des revendications précédentes, dotée d'au moins un segment qui présente une couche partielle avec un matériau biocompatible tel que le titane, le tantale, le niobium, l'or, le platine, le palladium ou un alliage d'au moins un de ces métaux.

9. Broche de connexion selon l'une des revendications précédentes, dans laquelle l'extrémité (13b) renforcée en épaisseur de la broche de connexion présente une section d'élargissement conçue sous forme de cylindre, de cône tronqué, ou de section d'une sphère, ou sous forme de prisme à quatre ou à plusieurs faces, ou de base de pyramide à quatre ou plusieurs faces, essentiellement en forme de tête de clou.

10. Passage (11) pour un appareil électromédical implantable (1) qui comprend au moins une broche de connexion (13a ; 13a') selon l'une des revendications précédentes.
